# EUROPEAN PATENT APPLICATION

(11) **EP 3 705 479 A1**
(43) Date of publication of application: **09.09.2020**
(21) Application number: 20160930.2
(22) Date of filing: 04.03.2020
(51) Int. Cl.: C07D 453/04, B01J 31/02

(54) **NEW AMIDE DERIVATIVES OF CINCHONA ALKALOIDS, METHOD FOR THE PREPARATION THEREOF AND USE THEREOF IN ASYMMETRIC PHASE TRANSFER REACTIONS**

(30) Priority: 05.03.2019 PL 42915019
(71) Applicant: Instytut Chemii Organicznej Polskiej Akademii Nauk, 01-224 Warszawa (PL)
(72) Inventor: Jurczak, Janusz, 02-384 Warszawa (PL); Majdecki, Maciej, 05-090 Raszyn (PL); Niedbala, Patryk, 05-090 Raszyn (PL)
(74) Representative: LDS Lazewski Depo & Partners

(57) **Abstract**

The object of the invention relates to a *Cinchona* alkaloid amide derivative of Formula **1** and Formula **3**. The invention also relates to methods for the preparation of the *Cinchona* alkaloid amide derivative of Formula **1** and Formula **3**, as well as its application as a catalyst in an asymmetric reaction under phase transfer conditions (PTC).

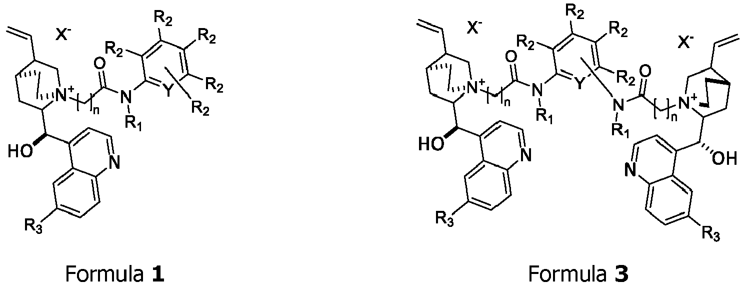

## Description

The invention relates to new amide derivatives of *Cinchona* alkaloids, the method of their preparation and their application in asymmetric organic chemistry. The amide derivatives according to the invention are useful as catalysts in reactions carried out under Phase-Transfer Catalysis (PTC) (liquid-liquid or liquid-solid), especially in the asymmetric variant for the Schiff base alkylation reaction.

Amide derivatives of *Cinchona* alkaloids which are catalysts in asymmetric PTC reactions as such, are known in state of the art. Efficiency and synthetic possibilities characterizing reactions carried out under phase transfer conditions cause that new catalysts used in such reactions are constantly sought after. Therefore, it is reasonable to introduce into the structure of catalysts groups that give the possibility of preorganizing the substrate molecule by forming intermolecular hydrogen bonds. The ideal choice in this case is the amide function, which is both a donor and acceptor of the created interactions. In particular, catalysts containing electron differentiating groups in their structure that change the electron density in the aromatic ring adjacent to the group capable of forming hydrogen bonds are desired. Such groups give the opportunity to influence the strength of hydrogen bonds created by changing the acidity of the proton attached to the nitrogen atom.

Despite the fact that several different classes of chiral PTC catalysts have been developed, their structures are largely limited to *Cinchona* alkaloids or binaphthyl and tartaric acid derivatives. A significant number of chiral catalysts constructed using the type of cinchonine (cinchonidine) or quinine (quinidine) type alkaloids (Formula A) were presented in the 1980s (Dolling, U.-H., et al., J. Am. Chem. Soc., 1984, 106, 446; Dolling, U.-H., et al., J. Org. Chem., 1987, 52, 4745).

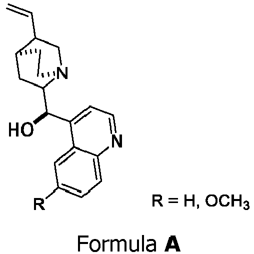

The size of *N-*substituents plays an important role here (Corey, EJ, et al., J. Am. Chem. Soc., 1997, 119, 12414; Lygo, B., et al., Tetrahedron Lett., 1997, 38, 8595). This subject was then further developed (Elango, S., Tetrahedron, 2005, 61, 1443; Najera, C., et al., Tetrahedron: Asymmetry, 2003, 14, 3705; Najera, C., et al., Tetrahedron: Asymmetry, 2002, 13, 2181). It has also been proven that *O*-substitution of quaternary salts provides the ability to affect the enantioselectivity of the catalysed reaction (Plaquevent, T., et al., Org. Lett., 2000, 2,2959; Palomo, C., et al., J. Am Chem. Soc., 2005, 127, 17622; Herrera, RP, et al., Angew. Chem. Int Ed., 2005, 44, 7975; Berkessel, A., et al., Chem. Eur. J., 2007, 13, 4483; Liu, Y., et al., Chem. Sci., 2011, 2, 1301; Provencher, BA, et al., Angew. Chem. Int. Ed., 2011, 50, 10565).

In order to improve the enantioselectivity of the reaction, among others in the asymmetric Sharpless dihydroxylation (Sharpless, K.B., et al., Chem. Rev., 1994, 94, 2483), a new class of dimeric catalysts has been developed (Jew, S.-S., Park, H .-G., et al., Chem. Commun., 2001, 1244; Jew, S.-S., Park, H.-G., et al., Tetrahedron Lett, 2001, 42, 4645; Jew, S.-S., Park, H.-G., et al., Angew. Chem. Int. Ed., 2002, 41, 3036; Jew, S.-S., Park, H.-G., et al., Eng. Chem. Int. Ed., 2005, 44, 1383).

This concept was then continued (Boratynski, P.J., Mol. Divers., 2015, 19, 385), also in the trimeric version (Siva, A., et al., J. Mol. Catal. A: Chem., 2006, 248, 1). In state of the art there are known *N-*substituted *Cinchona* alkaloid derivatives used in PTC reactions (Formula B), which, however, lack said groups capable of directional preorganization of the substrate (O'Donnell, M.J., Esikova, I., WO 95/06029, 1995).

Corey *et al.* disclosed further derivatives by introducing a 9-methyl anthracene substituent (Formula C) into the compound structure (Corey, E.J., et al., J. Am. Chem. Soc., 1998, 120, 13000-13001). In state of the art there are reports regarding model amide derivatives of *Cinchona* alkaloids used in chemotherapeutic studies (Formula D) (Jacobs, W.A., Heidelberger, M., J. Am. Chem. Soc., 1919, 41, 2090). In addition, phase transfer catalysts having in their structure two cinchonidine units connected with a polyethylene linker (Wang, X., Liang, Y., Yang, T., Wang, Y., Tetrahedron: Asymmetry, 2006, 18, 108-114) were presented.

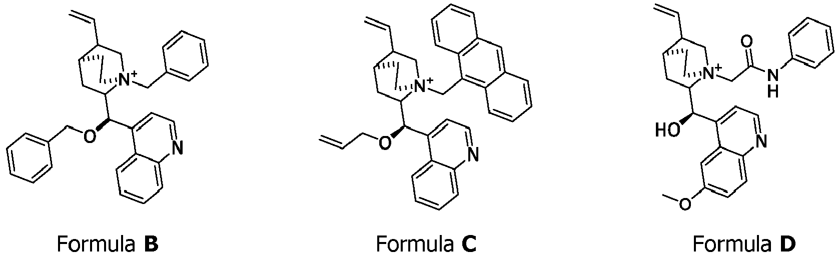

The object of the present invention is to provide chemical compounds being an amide derivatives of *Cinchona* alkaloids suitable for use as a catalyst in an asymmetric phase transfer reaction (PTC), which can be obtained from commercially available cheap substrates and can be used in low concentrations (about 5 mol% or less).

A further object is to provide the *Cinchona* alkaloid amide derivatives of the invention applicable as a catalyst in reactions under phase transfer conditions (liquid-liquid or liquid-solid), particularly in the asymmetric variant for the alkylation reaction of Schiff bases involving imine derivatives of a-amino acids as substrates, which leads to both high enantiomeric excess and high reaction yields.

A further object is to provide a process for the synthesis of *Cinchona* alkaloid amide derivatives according to the invention.

The above problems were solved by new amide derivatives of *Cinchona* alkaloids of general Formulas 1 and 3 having electron differentiating moieties in the aromatic ring attached to the nitrogen atom.

The invention provides a *Cinchona* alkaloid amide derivative of general Formula 1: 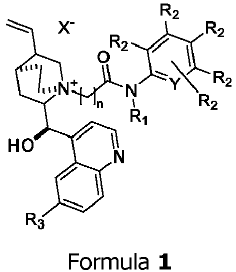 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**R₃** is independently hydrogen or methoxy (-OCH₃) group;
**X⁻** is any inorganic anion;
**Y** is nitrogen or CH group.
In a further aspect, the invention provides a *Cinchona* alkaloid amide derivative of general Formula 1, wherein:
**n** is an integer ranging from 1 to 2;
**R₁** is hydrogen or C₁-C₅ alkyl;
**R₂** is hydrogen, C₁-C₁₂ alkyl or nitro group;
**R₃** is hydrogen;
**X⁻** is Br⁻;
**Y** is CH group.

In a further aspect, the invention provides a *Cinchona* alkaloid amide derivative of Formula 1b:

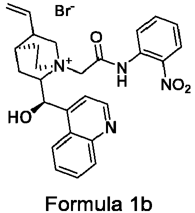

In a further aspect, the invention provides a *Cinchona* alkaloid amide derivative of Formula 1c:

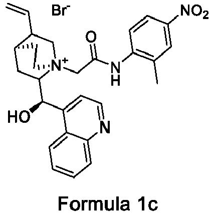

In a further aspect, the invention provides a process for the preparation of the *Cinchona* alkaloid amide derivative of general Formula 1 according to the invention, characterized in that it comprises reacting no more than two molar equivalents of the compound of general Formula 2: 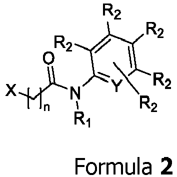 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**X** is Cl or Br;
**Y** is nitrogen or CH group;
with a *Cinchona* alkaloid selected from the group of cinchonine, cinchonidine, quinine or quinidine, wherein the reaction is carried out for 1 to 5 hours in an aprotic solvent at reflux.
In a further aspect, the invention provides a process for the preparation of the *Cinchona* alkaloid amide derivative of general Formula 1 according to the invention, characterized in that it comprises reacting no more than two molar equivalents of the compound of general Formula 2: 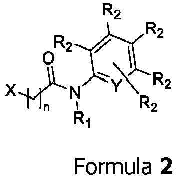 wherein:
**n** is an integer ranging from 1 to 2;
**R₁** is independently hydrogen or C₁-C₅ alkyl;
**R₂** is independently hydrogen, C₁-C₁₂ alkyl or nitro group;
**X** is Br;
**Y** is CH group;
with a *Cinchona* alkaloid selected from the group of cinchonine, cinchonidine, quinine or quinidine, wherein the reaction is carried out for 1 to 5 hours in an aprotic solvent at reflux.
In a further aspect, the invention provides a process for the preparation of the *Cinchona* alkaloid amide derivative of general Formula 1 according to the invention, characterized in that the aprotic solvent is THF or acetone.
In a further aspect, the invention provides a method for the preparation of the *Cinchona* alkaloid amide derivative of general Formula 1 according to the invention, characterized in that the reaction is carried out in the presence of one molar equivalent of the compound of general Formula 2.
In a further aspect, the invention provides a method for the preparation of the *Cinchona* alkaloid amide derivative of general Formula 1 according to the invention, characterized in that the reaction is carried out under an inert atmosphere.
In a further aspect, the invention provides a *Cinchona* alkaloid amide derivative of general Formula 3: 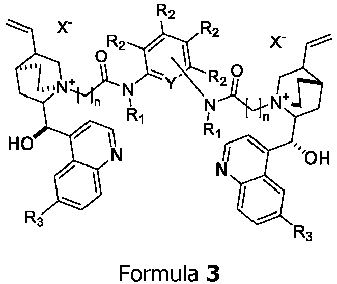 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**R₃** is independently hydrogen or methoxy (-OCH₃) group;
**X⁻ is** any inorganic anion;
**Y** is nitrogen or CH group.
In a further aspect, the invention provides a *Cinchona* alkaloid amide derivative of general Formula 3, wherein:
**n** is an integer ranging from 1 to 2;
**R₁** is hydrogen or C₁-C₅ alkyl;
**R₂** is hydrogen, C₁-C₁₂ alkyl or nitro group;
**R₃** is hydrogen;
**X⁻** is Br⁻;
**Y** is CH group.
In a further aspect, the invention provides a *Cinchona* alkaloid amide derivative of Formula 3b:

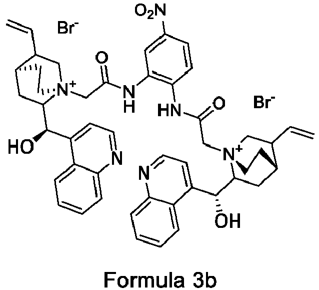

In a further aspect, the invention provides a *Cinchona* alkaloid amide derivative of Formula 3c:

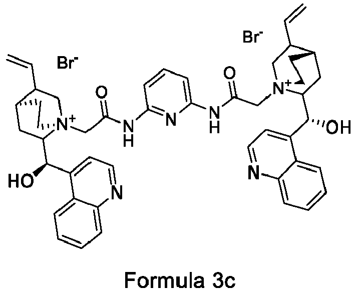

In a further aspect, the invention provides a process for the preparation of the *Cinchona* alkaloid amide derivative of general Formula 3 according to the invention, characterized in that it comprises reacting no more than one molar equivalent of the compound of general Formula 4: 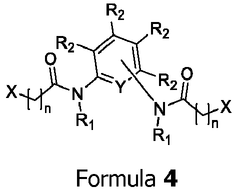 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**X** is Cl or Br;
**Y** is nitrogen or CH group;
with a *Cinchona* alkaloid selected from the group of cinchonine, cinchonidine, quinine or quinidine, wherein the reaction is carried out for 1 to 5 hours in an aprotic solvent at reflux.
In a further aspect, the invention provides a process for the preparation of the *Cinchona* alkaloid amide derivative of general Formula 3 according to the invention, characterized in that it comprises reacting no more than one molar equivalent of the compound of general Formula 4: 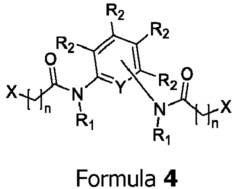 wherein:
**n** is an integer ranging from 1 to 2;
**R₁** is independently hydrogen or C₁-C₅ alkyl;
**R₂** is independently hydrogen, C₁-C₁₂ alkyl or nitro group;
**X** is Br;
**Y** is CH group;
with a *Cinchona* alkaloid selected from the group of cinchonine, cinchonidine, quinine or quinidine, wherein the reaction is carried out for 1 to 5 hours in an aprotic solvent at reflux.
In a further aspect, the invention provides a process for the preparation of the *Cinchona* alkaloid amide derivative of general Formula 3 according to the invention, characterized in that the aprotic solvent is THF or acetone.

In a further aspect, the invention provides a method for the preparation of the *Cinchona* alkaloid amide derivative of general Formula 3 according to the invention, characterized in that the reaction is carried out in the presence of 0.5 molar equivalent of the compound of general Formula 4.
In a further aspect, the invention provides a method for the preparation of the *Cinchona* alkaloid amide derivative of general Formula 3 according to the invention, characterized in that the reaction is carried out under an inert atmosphere.
In a further aspect, the invention provides the use of the *Cinchona* alkaloid amide derivative of the invention as a catalyst in an asymmetric reaction under phase transfer conditions (PTC). Preferably the reaction is an asymmetric Schiff base alkylation reaction involving imine derivatives of a-amino acids esters as substrates. More preferably, the reaction proceeds under liquid-liquid or liquid-solid phase transfer conditions.
All technical and scientific terms used in this document have the meaning as commonly understood by person skilled in the art.
The compounds disclosed herein have stereogenic carbon centers. Such compounds include racemic mixtures of all stereoisomers, including enantiomers, diastereomers and atropisomers. Both racemic and diastereomeric mixtures and all individual optical isomers isolated and synthesized, substantially optically pure, are within the scope of the present invention.
The invention includes all any stereochemical forms, including enantiomeric or diastereomeric, and geometric isomers of the compounds disclosed herein, or mixtures thereof.
The salts used herein include salts with any inorganic anion.

### Examples

The object of the invention in the examples below is shown in Table 1, in which representative structures of the compounds of the invention are presented together with spectral characteristics (mass spectrometry). The *Cinchona* alkaloid amide derivatives set out below do not limit the scope of the invention as defined in the claims, and are only representative embodiments of the present invention.

**Table 1.**

| | |
|---|---|
| 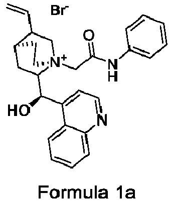 | 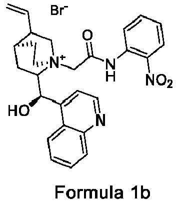 |
| HRMS ESI (m/z): | HRMS ESI (m/z): |
| calc. for: C₂₇H₃₀N₃O₂ [M]⁺: 428.2338, | calc. for: C₂₇H₂₉N₄O₄ [M]⁺: 473.2189, |
| found: 428.2336. | found: 473.2177. |
| 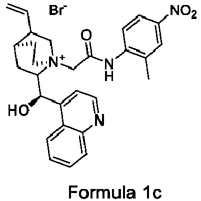 | 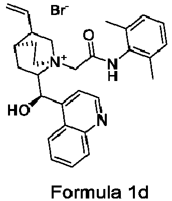 |
| HRMS ESI (m/z): | HRMS ESI (m/z): |
| calc. for: C₂₈H₃₁N₄O₄ [M]⁺: 487.2345, | calc. for: C₂₉H₃₄N₃O₂ [M]⁺: 456.2651, |
| found: 487.2333. | found: 456.2640. |
| 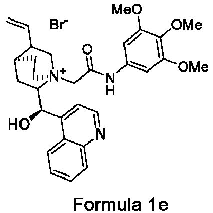 | 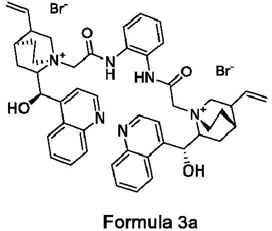 |
| HRMS ESI (m/z): | HRMS ESI (m/z): |
| calc. for: C₃₀H₃₆N₃O₅ [M]⁺: 518.2655, | calc. for: C₄₈H₅₄N₆O₄ [M]²⁺: 389.2098, |
| found: 518.2646. | found: 389.2117. |
| 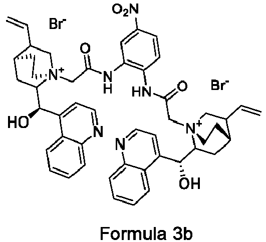 | 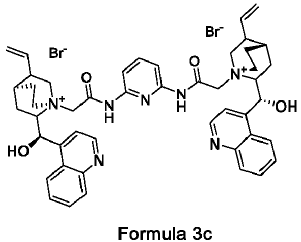 |
| HRMS ESI (m/z): | HRMS ESI (m/z): |
| calc. for: C₄₈H₅₃N₇O₆ [M]²⁺: 411.7023, | calc. for: C₄₇H₅₃N₇O₄ [M]²⁺: 389.7074, |
| found: 411.7026. | found: 389.7076. |

The object of the invention in the following embodiments is shown in Examples 1-8, which show the process for the preparation of *Cinchona* alkaloid amide derivatives according to the invention.

The object of the invention in the following embodiments is shown in Tables 2 and 3 and in Examples 9 and 10, in which the values of yield and enantiomeric excesses for the alkylation reaction of Schiff bases carried out in the presence of amide derivatives of the *Cinchona* alkaloids of the invention as catalysts are presented.

### Example 1. Two-step synthesis of the compound of Formula 1a: N-(N'-phenylacetamide)cinchonidine bromide

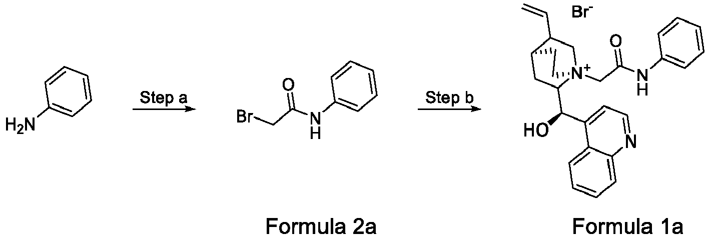

### Step a

Aniline (1.0 g, 10.7 mmol) was dissolved in 15 mL of dichloromethane, followed by the addition of a solution of K₂CO₃ (2.23 g, 16.1 mmol) in water (20 mL). The reaction mixture was cooled to 0 °C and a solution of bromoacetyl bromide (3.24 g, 16.1 mmol, 1.4 mL) in dichloromethane (3.6 mL) was slowly added dropwise. The reaction was controlled by TLC for about one hour, after which the phases were separated, and the aqueous phase was extracted with dichloromethane (3 x 20 mL). The combined organic phases were dried over anhydrous Na₂SO₄ and the solvent was evaporated under reduced pressure to give the product 2.1 g (92%) as a colorless solid (mp 132-133 °C).
¹H NMR (400 MHz, DMSO) δ 10.35 (s, 1H), 7.59 (d, J = 7.9 Hz, 2H), 7.33 (t, J = 7.9 Hz, 2H), 7.08 (t, J = 7.4 Hz, 1H), 4.04 (s, 2H).
¹³C NMR (100 MHz, DMSO) δ 164.7, 138.6, 128.8, 123.8, 119.2, 30.4.

### Step b

To a solution of cinchonidine (1.0 g, 3.4 mmol) in tetrahydrofuran (30 mL) amide **2a** was added (0.73 g, 3.4 mmol). The mixture was refluxed for about 1.5 hours under TLC control. Subsequently, the solvent was evaporated under reduced pressure, and the dry residue was dissolved in dichloromethane (2 mL). The catalyst solution prepared in this way was slowly added dropwise to diethyl ether (15 mL). The resulting precipitate was filtered off, washed with diethyl ether and then dried to give the product 1.66 g (96%) as a cream solid (mp 152-153 °C).
¹H NMR (400 MHz, DMSO) δ 11.17 (s, 1H), 8.96 (d, J = 3.4 Hz, 1H), 8.17 (d, J = 8.0 Hz, 1H), 8.06 (d, J = 8.1 Hz, 1H), 7.86-7.71 (m, 4H), 7.54 (t, J = 7.1 Hz, 1H), 7.44 (t, J = 7.2 Hz, 2H), 7.19 (t, J = 6.8 Hz, 1H), 6.75 (s, 1H), 6.16 (s, 1H), 5.75-5.60 (m, 1H), 5.20 (d, J = 17.3 Hz, 1H), 4.96 (dd, J = 24.1, 13.1 Hz, 2H), 4.78 (d, J = 15.6 Hz, 1H), 4.44 (bs, 1H), 4.37 (d, J = 12.1 Hz, 1H), 4.28 (t, J = 8.2 Hz, 1H), 4.00 (t, J = 11.1 Hz, 1H), 3.82 (t, J = 9.3 Hz, 1H), 2.87 (bs, 1H), 2.17-1.93 ( m, 4H), 1.17 (t, J = 10.2 Hz, 1H).
¹³C NMR (100 MHz, DMSO) δ 162.6, 150.1, 147.5, 144.8, 138.1, 137.7, 129.9, 129.4, 129.0, 127.0, 124 , 6, 124.1, 122.9, 120.1, 119.7, 115.9, 65.0, 64.7, 60.6, 59.4, 55.5, 36.9, 25.3 , 24.7, 20.8.

### Example 2. Two-step synthesis of the compound of Formula 1b: N-(N'-2-nitro-phenylacetamide)cinchonidine bromide

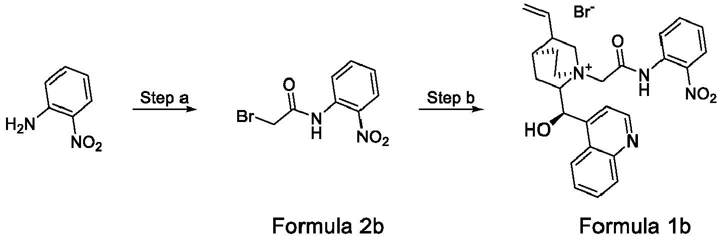

Two-step synthesis of the compound of Formula **1b** was carried out as in Example 1. Amide **2b** 2.7 g (97%) was obtained as a cream solid (mp 62-63 °C).
¹H NMR (400 MHz, DMSO) δ 10.67 (s, 1H), 8.00 (dd, J = 8.2, 0.8 Hz, 1H), 7.76-7.69 (m, 2H), 7.41 (ddd, J = 8.5, 6.5, 2.3 Hz, 1H), 4.14 (s, 2H).
¹³C NMR (100 MHz, DMSO) δ 165.1, 142.2, 134.1, 130.6, 125.8, 125.2, 125.0, 29.4.

The compound of Formula **1b** 1.83 g (97%) was obtained as a yellowish solid (mp 165-167 °C (decomposition)).
¹H NMR (400 MHz, DMSO) δ 11.39 (s, 1H), 8.97 (d, J = 4.5 Hz, 1H), 8.15-8.05 (m, 3H), 7.89-7.82 (m, 1H), 7.81-7.75 (m, 2H), 7.70 (d, J = 7.2 Hz, 1H), 7.59-7.51 (m, 2H), 6.83 (d, J = 3.7 Hz, 1H), 6.13 (s, 1H), 5.71-5.62 (m, 1H), 5.16 (d, J = 17, 4 Hz, 1H), 4.98-4.88 (m, 2H), 4.73 (d, J = 16.1 Hz, 1H), 4.50-4.36 (m, 1H), 4.29 (t, J = 9.2 Hz, 1H), 4.11 (d, J = 12.5 Hz, 1H), 4.00-3.91 (m, 1H), 3.74 (t, J = 10.6 Hz, 1H), 2.87 (bs, 1H), 2.16-2.06 (m, 2H), 2.03-1.93 (m, 2H), 1.09 (t, J = 11.1 Hz, 1H).
¹³C NMR (100 MHz, DMSO) δ 163.3, 150.1, 147.5, 144.8, 143.4, 137.9, 134.3, 129.7, 129.4, 129.0, 127.0 (x2), 126.3, 125.2, 124.2, 123.3, 120.0, 115.8, 65.2, 64.9, 60.3, 59.0, 55.7, 36.8, 25.2, 24.7, 21.0.

### Example 3. Two-step synthesis of the compound of Formula 1c: N-(N'-4-nitro-o-toluidineacetamide)cinchonidine bromide

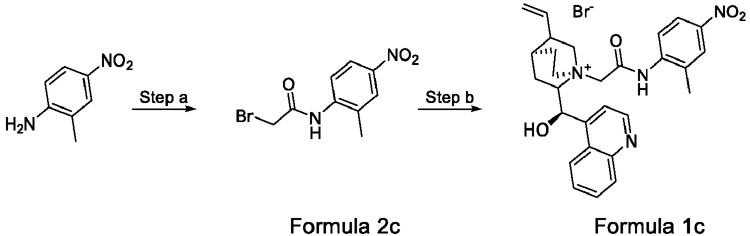

Two-step synthesis of the compound of Formula **1c** was carried out as in Example 1. Amide **2c** 2.97 g (98%) was obtained as a colorless solid (mp 129-130 °C).
¹H NMR (400 MHz, DMSO) δ 9.93 (s, 1H), 8.14 (d, J = 2.3 Hz, 1H), 8.08 (dd, J = 8.9, 2.6 Hz, 1H), 7.90 (d, J = 8.9 Hz, 1H), 4.20 (s, 2H), 2.36 (s, 3H).
¹³C NMR (100 MHz, DMSO) δ 165.6, 143.6, 142.1, 131.6, 125.4, 123.5, 121.7, 29.9, 17.7.

The compound of Formula **1c** 1.9 g (98%) was obtained as a cream solid (mp 161-162 °C).
¹H NMR (400 MHz, DMSO) δ 11.04 (s, 1H), 8.97 (d, J = 3.6 Hz, 1H), 8.09 (dd, J = 24.7, 8.2 Hz , 2H), 7.90 (d, J = 7.8 Hz, 1H), 7.83-7.67 (m, 3H), 7.51 (dd, J = 16.5, 8.1 Hz, 2H), 6.91 (d, J = 2.2 Hz, 1H), 6.12 (s, 1H), 5.71-5.57 (m, 1H), 5.10 (dd, J = 40 , 3, 16.7 Hz, 2H), 4.88 (dd, J = 24.6, 13.4 Hz, 2H), 4.49 (t, J = 6.2 Hz, 1H), 4.34 (t, J = 8.5 Hz, 1H), 4.14 (d, J = 11.3 Hz, 1H), 4.00 (t, J = 11.0 Hz, 1H), 3.78 (t, J = 9.5 Hz, 1H), 2.88 (bs, 1H), 2.45 (s, 3H), 2.09 (bs, 2H), 2.03-1.90 (m, 2H), 1.05 (t, J = 10.3 Hz, 1H).
¹³C NMR (100 MHz, DMSO) δ 163.6, 150.1, 147.5, 146.2, 144.8, 137.9, 137.2, 135.5, 129.8, 129.4, 127.7, 126.9 (x2), 124.2, 123.2, 122.6, 119.9, 115.7, 65.3, 64.5, 60.1, 58.6, 55.8, 36.7, 25.2, 24.7, 21.2, 18.1.

### Example 4. Two-step synthesis of the compound of Formula 1d: N-(N'2,6-dimethylphenylacetamide)cinchonidine bromide

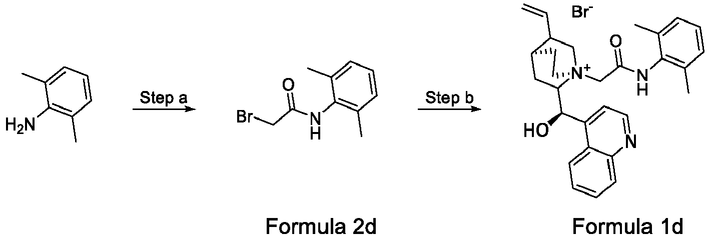

Two-step synthesis of the compound of Formula **1d** was carried out as in Example 1. Amide **2d** 2.6 g (99%) was obtained as a colorless solid (mp 150-151 °C).
¹H NMR (400 MHz, DMSO) δ 9.68 (s, 1H), 7.12-7.04 (m, 3H), 4.05 (s, 2H), 2.15 (s, 6H).
¹³C NMR (100 MHz, DMSO) δ 164.7, 135.1, 134.2, 127.7, 126.7, 29.3, 17.8,

The compound of Formula **1d** 1.77 g (97%) was obtained as a colorless solid (mp 217-218 °C).
¹H NMR (400 MHz, DMSO) δ 10.45 (s, 1H), 8.97 (d, J = 4.5 Hz, 1H), 8.22 (d, J = 8.5 Hz, 1H), 8.06 (d, J = 8.4 Hz, 1H), 7.82-7.73 (m, 2H), 7.50 (t, J = 7.6 Hz, 1H), 7.20-7.11 (m, 3H), 6.88 (d, J = 4.2 Hz, 1H), 6.16 (bs, 1H), 5.72-5.60 (m, 1H), 5.14 (dd, J = 28.9, 16.7 Hz, 2H), 4.89 (t, J = 14.7 Hz, 2H), 4.49 (t, J = 8.6 Hz, 1H), 4.40 (t, J = 9.2 Hz, 1H), 4.28 (d, J = 12.4 Hz, 1H), 4.05 (t, J = 11.4 Hz, 1H), 3.83 (t, J = 10.7 Hz, 1H), 2.89 (bs, 1H), 2.25 (s, 6H), 2.10 (bs, 2H), 2.02-1.92 (m, 2H), 1.09 (t, J = 10.9 Hz, 1H).
¹³C NMR (100 MHz, DMSO) δ 162.9, 150.1, 147.5, 144.8, 138.0, 135.1, 133.3, 129.8, 129.4, 127.9, 127.2, 126.9, 124.2, 123.2, 120.0, 115.7, 65.3, 64.4, 60.0, 58.5, 55.5, 36.8, 25.3, 24.7, 21.0, 18.3.

### Example 5. Two-step synthesis of the compound of Formula 1e: N-(N'3,4,5-trimethoxyphenylacetamide)cinchonidine bromide

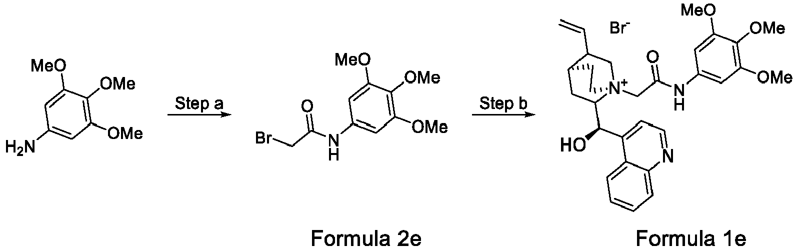

Two-step synthesis of the compound of Formula **1e** was carried out as in Example 1. Amide **2e** 3.1 g (94%) was obtained as a colorless solid (mp 125-126 °C).
¹H NMR (400 MHz, DMSO) δ 10.29 (s, 1H), 6.96 (s, 2H), 4.01 (s, 2H), 3.75 (s, 6H), 3.62 (s, 3H),
¹³C NMR (100 MHz, DMSO) δ 164.5, 152.7, 134.7, 133.8, 97.0, 60.1, 55.7, 30.4.

The compound of Formula **1e** 1.9 g (95%) was obtained as a cream solid (mp 147-148 °C).
¹H NMR (400 MHz, DMSO) δ 11.07 (s, 1H), 8.97 (d, J = 4.1 Hz, 1H), 8.21 (d, J = 8.3 Hz, 1H), 8.07 (d, J = 8.3 Hz, 1H), 7.84-7.75 (m, 2H), 7.54 (t, J = 7.4 Hz, 1H), 7.16 (s , 2H), 6.75 (d, J = 2.7 Hz, 1H), 6.16 (s, 1H), 5.79-5.64 (m, 1H), 5.19 (d, J = 17.3 Hz, 1H), 4.93 (t, J = 12.7 Hz, 2H), 4.72 (d, J = 15.6 Hz, 1H), 4.41 (s, 1H), 4.27 (t, J = 11.3 Hz, 2H), 4.01 (t, J = 11.4 Hz, 1H), 3.84 (s, 1H), 3.80 (s, 6H), 3.66 (s, 3H), 2.87 (s, 1H), 2.07 (dd, J = 36.1, 12.5 Hz, 4H), 1.20 (t, J = 9.7 Hz, 1H).
¹³C NMR (100 MHz, DMSO) δ 162.4, 152.9, 150.1, 147.6, 144.8, 138.1, 134.5, 133.8, 129.9, 129.4, 126.9, 124.2, 123.0, 120.0, 116.1, 97.6, 65.1, 65.0, 60.8, 60.2, 59.5, 55.9, 55.4, 37.0, 25.3, 24.7, 20.7.

### Example 6. Two-step synthesis of the compound of Formula 3a: N,N'-(1,2-phenylene)bis(2-cinchonidineacetamide) dibromide

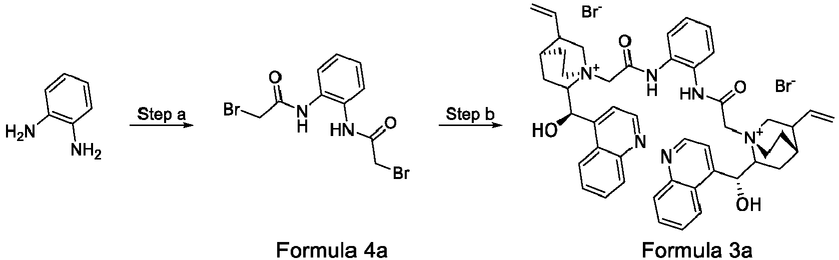

### Step a

1,2-Diaminobenzene (1.0 g, 9.2 mmol) was dissolved in 20 mL of dichloromethane, followed by the addition of a solution of K₂CO₃ (3.83 g, 27.7 mmol) in water (25 mL). The reaction mixture was cooled to 0 °C and a solution of bromoacetyl bromide (5.6 g, 27.7 mmol, 2.4 mL) in dichloromethane (2.6 mL) was slowly added dropwise. The reaction was controlled by TLC for about one hour, after which the resulting precipitate was filtered off, washed with distilled water and diethyl ether, and then dried under reduced pressure. The product 2.8 g (87%) was obtained as a colorless solid (mp 175-176 °C (decomposition)).
¹H NMR (400 MHz, DMSO) δ 9.71 (s, 2H), 7.53 (dd, J = 5.7, 3.6 Hz, 2H), 7.21 (dd, J = 6.0, 3.5 Hz, 2H), 4.12 (s, 4H).
¹³C NMR (100 MHz, DMSO) δ 165.2, 130.3, 125.6, 124.9, 30.2.

### Step b

To a solution of cinchonidine (1.0 g, 3.4 mmol) in tetrahydrofuran (50 mL) diamide **4a** (0.60 g, 1.7 mmol) was added. The mixture was refluxed for about three hours under TLC control. After this time, the solvent was evaporated under reduced pressure, suspended in acetone/diethyl ether [1:1, v/v] sonicated for five minutes. The resulting precipitate was filtered off, washed with diethyl ether and dried under reduced pressure. The product 1.36 g (85%) was obtained as a colorless solid (mp 209-210 °C).
¹H NMR (400 MHz, DMSO) δ 10.58 (s, 2H), 8.95 (d, J = 3.3 Hz, 2H), 8.28 (d, J = 8.0 Hz, 2H), 8.05 (d, J = 8.1 Hz, 2H), 7.84 (s, 2H), 7.81-7.70 (m, 4H), 7.55 (t, J = 6.9 Hz, 2H), 7.40 (bs, 2H), 6.66 (d, J = 2.6 Hz, 2H), 6.27 (s, 2H), 5.67-5.55 (m, 2H), 5.22 (dd, J = 45.5, 16.3 Hz, 4H), 4.93 (dd, J = 58.5, 12.8 Hz, 4H), 4.45 (bs, 2H), 4.27 (d, J = 8.9 Hz, 4H), 4.09 (t, J = 11.0 Hz, 2H), 3.87 (t, J = 9.9 Hz, 2H), 2.83 (bs, 2H), 2.03-1.94 (m, 4H), 1.14 (t, J = 9.6 Hz, 2H).
¹³C NMR (100 MHz, DMSO) δ 163.2, 150.1, 147.5, 144.8, 137.9, 129.8, 129.5, 129.3, 127.1, 126.2, 125.6, 124.1, 123.3, 120.0, 115.9, 65.3, 65.0, 60.5, 59.7, 55.1, 36.8, 25.2, 24.6 , 20.8.

### Example 7. Two-step synthesis of the compound of Formula 3b: N,N'-(4-nitro-1,2-phenylene)bis(2-cinchonidineacetamide) dibromide

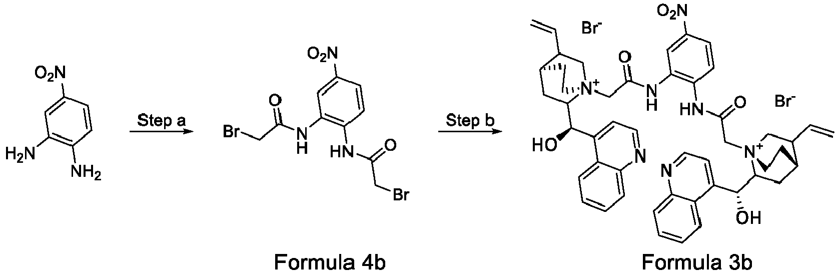

The two-step synthesis of the compound of Formula **3b** was carried out as in Example 6. Diamide **4b** 3.1 g (85%) was obtained as a yellowish solid (mp 152-153 °C).
¹H NMR (400 MHz, DMSO) δ 10.09 (s, 1H), 10.07 (s, 1H), 8.42 (d, J = 2.5 Hz, 1H), 8.09 (dd, J = 9.0, 2.6 Hz, 1H), 7.96 (d, J = 9.0 Hz, 1H), 4.19 (s, 2H), 4.17 (s, 2H).
¹³C NMR (100 MHz, DMSO) δ 165.7, 165.6, 143.5, 136.5, 129.8, 124.3, 120.8, 119.9, 30.1 (x2).

The compound of Formula **3b** 1.64 g (98%) was obtained as a yellowish solid (mp 211-212 °C (decomposition)).
¹H NMR (400 MHz, DMSO) δ 10.94 (d, J = 12.3 Hz, 2H), 8.96 (dd, J = 4.4, 2.3 Hz, 2H), 8.77 (d , J = 2.3 Hz, 1H), 8.34-8.22 (m, 4H), 8.05 (d, J = 8.6 Hz, 2H), 7.81-7.71 (m, 4H), 7.59 (dd, J = 15.7, 7.7 Hz, 2H), 6.66 (s, 2H), 6.31 (d, J = 11.8 Hz, 2H), 5.74-5.59 (m, 2H), 5.34 (dd, J = 36.0, 15.7 Hz, 2H), 5.18 (dd, J = 17.2, 10.1 Hz, 2H), 5.04 (dd, J = 15.6, 5.1 Hz, 2H), 4.89 (d, J = 10.3 Hz, 2H), 4.46 (bs, 2H), 4.34-4.17 (m, 4H), 4.11 (dd, J = 18.5, 11.2 Hz, 2H), 3.87 (dd, J = 20.6, 9.7 Hz, 2H), 2.85 (bs, 2H), 2.15-1.98 (m, 8H), 1.22-1.13 (m, 2H).
¹³C NMR (100 MHz, DMSO) δ 172.6, 163.7, 150.1, 147.5, 144.8, 144.7, 144.1, 141.2, 137.9, 129.8, 129.3, 127.2, 127.1, 124.1, 123.3, 120.8, 120.0, 116.0, 65.6, 65.0, 60.6, 59.7, 55.0, 36.9, 25.3, 24.7, 20.8.

### Example 8. Two-step synthesis of the compound of Formula 3c: N,N'-(pyridin-2,6-diyl) bis(2-cinchonidineacetamide) dibromide

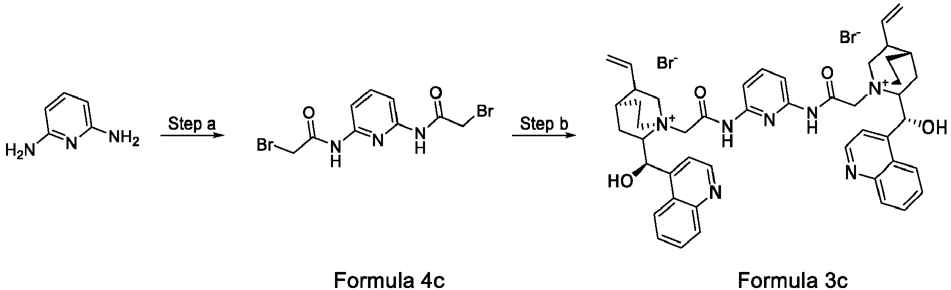

Two-step synthesis of the compound of Formula **3c** was carried out as in Example 6. Diamide **4c** 2.87 g (89%) was obtained as a cream solid (mp 140-141 °C (decomposition)).
¹H NMR (400 MHz, DMSO) δ 10.50 (s, 2H), 7.85-7.69 (m, 3H), 4.17 (s, 4H).
¹³C NMR (100 MHz, DMSO) δ 165.7, 149.8, 140.5, 109.6, 30.2.

The reaction was carried out in acetone. The compound of Formula **3c** 1.5 g (93%) was obtained as a yellowish solid (mp 204-205 °C).
¹H NMR (400 MHz, DMSO) δ 11.16 (s, 1H), 8.97 (d, J = 4.0 Hz, 1H), 8.18-8.04 (m, 4H), 7.84-7.76 (m, 2H), 7.59 (t, J = 6.0 Hz, 1H), 6.77 (s, 1H), 6.15 (s, 1H), 5.79-5.64 (m, 1H), 5.15 (dd, J = 35.2, 16.7 Hz, 2H), 4.92 (dd, J = 25.1, 13.1 Hz, 2H), 4.45 (bs, 1H), 4.29 (bs, 2H), 4.04 (t, J = 10.7 Hz, 1H), 3.82 (bs, 1H), 2.88 (bs, 1H), 2.16-1.96 (m, 4H), 1.19 (t, J = 9.3 Hz, 1H).
¹³C NMR (100 MHz, DMSO) δ 164.0, 150.2, 149.3, 147.6, 144.8, 141.2, 138.1 (x2), 130.0, 129.4, 127.0, 124.2, 122.9, 120.1, 115.9, 65.0 (x2), 60.6, 59.4, 55.5, 36.9, 25.3, 24.7, 20.8.

### Example 9. The application of Cinchona alkaloid amide derivatives of Formula 1a, 1b, 1c, 1d, 1e, 3a, 3b, 3c as catalysts for asymmetric Schiff base alkylation reactions involving imine derivatives of a-amino acids as substrates.

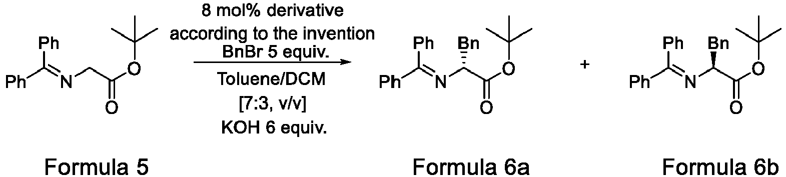

The *Cinchona* alkaloid amide derivative of the invention (0.016 mmol) and the *N-*(diphenylmethylene) glycine *tert*-butyl ester **5** (59 mg, 0.2 mmol) were dissolved in toluene/ dichloromethane [7:3, v/v] (1 mL). Then benzyl bromide (0.6 mmol, 72 µl) and 50% KOH aqueous solution (0.6 mL) were added. The reaction was carried out at 0 °C for 8-12 hours (TLC control). After the reaction was completed, the solvents were evaporated under reduced pressure, and the dry residue was purified by column chromatography using hexane/ethyl acetate [99:1 → 95:5, v/v] as eluent to give the desired product as a colorless oil. The results are shown in Table 2.

**Table 2. Summary of results obtained for the asymmetric alkylation reaction of Schiff bases with compound 5 as a substrate and the corresponding derivative of the invention as a catalyst.**

| Entry | Derivative according to the invention, Formula No. | The C₉ carbon configuration of the compound of Formula | Stereochemistry of the major enantiomer | ee [%] | Yield [%] |
|---|---|---|---|---|---|
| 1 | 1a | *R* | *S* | 31 | 86 |
| 2 | 1b | *R* | *S* | 74 | 97 |
| 3^{a} | 1b | *R* | *S* | 73 | 91 |
| 4^{b} | 1b | *R* | *S* | 72 | 95 |
| 5 | 1c | *R* | *S* | 73 | 97 |
| 6 | 1d | *R* | *S* | 67 | 95 |
| 7 | 1e | *R* | *S* | 63 | 81 |
| 8 | 3a | *R* | *S* | 30 | 91 |
| 9 | 3b | *R* | *S* | 80 | 98 |
| 10 | 3c | *R* | *S* | 78 | 97 |

| | | | | | |
|---|---|---|---|---|---|
| Enantiomeric excess was determined by chiral high performance liquid chromatography (HPLC) using a Daicel Chiralcel OD-H column, as the eluent using hexane/isopropanol mixture [99.5:0.5, v/v], flow = 0.5 mL/min, with detection (UV-DAD) at λ = 254 nm. Retention times: 19.6 min (R) and 30.2 min (S). ^{a} Reaction carried out at 0 °C with 5 mol% catalyst. ^{b} Reaction carried out at room temperature using solid KOH (3 molar equiv.). | | | | | |

### Example 10. The application of a Cinchona alkaloid amide derivative of the invention of Formula 1b as a catalyst for asymmetric alkylation with substituted benzyl bromides.

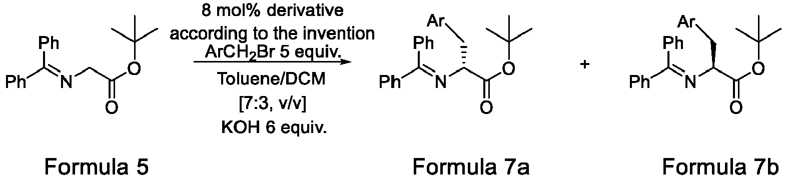

For the *Cinchona* alkaloid amide derivative of the invention of Formula **1b,** the reaction was carried out according to the procedure of Example 9, using a series of substituted benzyl bromides as the alkylating agents. The results of enantiomeric excess and yields are presented in Table 3.

**Table 3. Summary of results obtained for asymmetric alkylation of Schiff bases using compound 5 as a substrate, a series of alkylating agents and a derivative of the invention of Formula 1b as a catalyst.**

| Entry | Alkylating agent | Major enantiomer | ee [%] | Yield [%] | Retention time [min] | |
|---|---|---|---|---|---|---|
| | | | | | *R* (Formula **7a**) | *S* (Formula **7b**) |
| 1 | BnBr | *S* | 74 | 98 | 19.6 | 30.2 |
| 2 | 2-F-PhCH₂Br | *S* | 88 | 95 | 19.7 | 23.0 |
| 3 | 2-Cl-PhCH₂Br | *S* | 76 | 97 | 25.4 | 28.3 |
| 4 | 3-Cl-PhCH₂Br | *S* | 53 | 94 | 18.9 | 29.2 |
| 5 | 4-Cl-PhCH₂Br | *S* | 82 | 93 | 13.3 | 20.8 |
| 6 | 2-Me-PhCH₂Br | *S* | 62 | 92 | 29.8 | 54.0 |
| 7 | 3-Me-PhCH₂Br | *S* | 66 | 94 | 24.5 | 35.9 |
| 8 | 4-Me-PhCH₂Br | *S* | 78 | 91 | 14.8 | 16.2 |

| | | | | | | |
|---|---|---|---|---|---|---|
| Enantiomeric excess was determined by chiral high performance liquid chromatography (HPLC) using a Daicel Chiralcel OD-H column as the eluent and using hexane/isopropanol mixture [99.5:0.5, v/v], flow = 0.5 mL/min, with detection (UV-DAD) at λ = 254 nm. | | | | | | |

Based on the results obtained, it was found that the newly developed and newly obtained amide derivatives of alkaloids according to the invention can be used as effective catalysts in asymmetric reactions under PTC conditions (Table 2), in particular showing a wide utility in relation to the used substrates (Table 3). The *Cinchona* alkaloid amide derivatives of the present invention find application as PTC catalysts in both liquid-liquid and liquid-solid conditions.

## Claims

1. A *Cinchona* alkaloid amide derivative of Formula **1:** 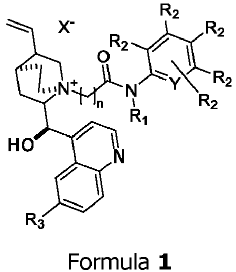 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**R₃** is independently hydrogen or methoxy (-OCH₃) group;
**X⁻** is any inorganic anion;
**Y** is nitrogen or CH group.

2. The *Cinchona* alkaloid amide derivative according to claim 1, wherein:
**n** is an integer ranging from 1 to 2;
**R₁** is hydrogen or C₁-C₅ alkyl;
**R₂** is hydrogen, C₁-C₁₂ alkyl or nitro group;
**R₃** is hydrogen;
**X⁻** is Br⁻;
**Y** is CH group.

3. The *Cinchona* alkaloid amide derivative according to claim 1, wherein the derivative is defined by the following Formula **1b** or Formula **1c:**

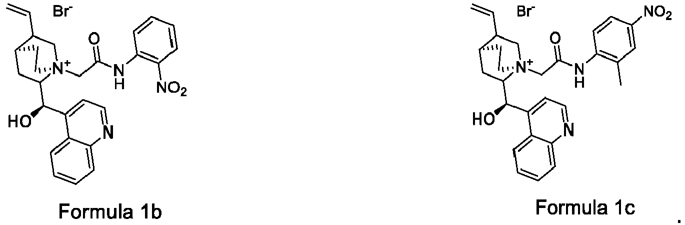

4. A method for the preparation of the *Cinchona* alkaloid amide derivative of any one of claims 1 to 3, **characterized in that** it comprises reacting no more than two molar equivalents of the compound of Formula **2:** 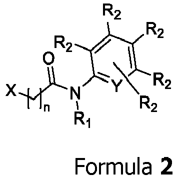 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**X** is Cl or Br;
**Y** is nitrogen or CH group;
with a *Cinchona* alkaloid selected from the group of cinchonine, cinchonidine, quinine or quinidine, wherein the reaction is carried out for 1 to 5 hours in an aprotic solvent at reflux.

5. The method according to claim 4, wherein the aprotic solvent is THF or acetone.

6. The method according to any of claims 4 or 5, wherein the reaction is carried out in the presence of one molar equivalent of the compound of Formula 2.

7. A *Cinchona* alkaloid amide derivative of Formula **3:** 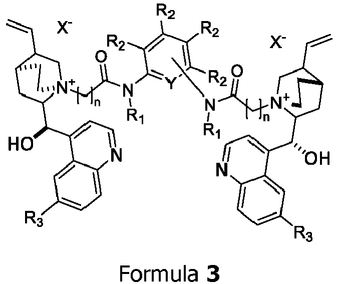 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**R₃** is independently hydrogen or methoxy (-OCH₃) group;
**X⁻** is any inorganic anion;
**Y** is nitrogen atom or CH group.

8. The *Cinchona* alkaloid amide derivative according to claim 7, wherein:
**n** is an integer ranging from 1 to 2;
**R₁** is hydrogen or C₁-C₅ alkyl;
**R₂** is hydrogen, C₁-C₁₂ alkyl or nitro group;
**R₃** is hydrogen;
**X⁻** is Br⁻;
**Y** is CH group.

9. The *Cinchona* alkaloid amide derivative according to claim 8, wherein the derivative is defined by the following Formula **3b** or Formula **3c:**

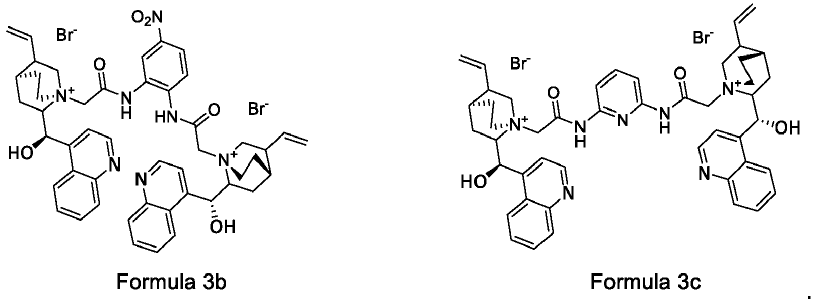

10. A method for the preparation of a *Cinchona* alkaloid amide derivative of Formula **3** according to any one of claims 7 to 9, **characterized in that** it comprises reacting no more than one molar equivalent of the compound of Formula **4:** 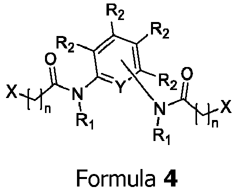 wherein:
**n** is an integer ranging from 1 to 5;
**R₁** is independently hydrogen, C₁-C₅ alkyl or C₅-C₁₆ aryl;
**R₂** is independently hydrogen, halogen, C₁-C₁₂ alkyl, C₅-C₁₆ aryl, C₁-C₅ branched alkyl, nitro group (-NO₂), trifluoromethyl group (-CF₃), nitrile group (-CN), alkoxy -OC₁-C₁₂ group;
**X** is Cl or Br;
**Y** is nitrogen or CH group;
with a *Cinchona* alkaloid selected from the group of cinchonine, cinchonidine, quinine or quinidine, wherein the reaction is carried out for 1 to 5 hours in an aprotic solvent at reflux.

11. The method according to claim 10, wherein the aprotic solvent is THF or acetone.

12. The method according to any one of claims 10 to 11, wherein the reaction is carried out in the presence of 0.5 molar equivalent of the compound of Formula **2.**

13. A use of the *Cinchona* alkaloid amide derivative according to any one of claims 1 to 3 or the *Cinchona* alkaloid amide derivative according to any one of claims 7 to 9 as a catalyst in an asymmetric reaction under phase transfer conditions (PTC).

14. The use according to claim 13, wherein said reaction is an asymmetric Schiff base alkylation reaction involving imine derivatives of a-amino acids esters as substrates.

15. The use according to claim 13 or 14, wherein the reaction proceeds under liquid-liquid or liquid-solid phase transfer conditions.
